(19) Europäisches Patentamt
European Patent Office
Office européen des brevets

(11) Publication number: **0 322 335 A1**

(12) **EUROPEAN PATENT APPLICATION**

(21) Application number: **88500031.5**

(22) Date of filing: **23.03.88**

(51) Int. Cl.[4]: **C 07 D 277/18**
C 07 D 487/04,
C 07 D 471/04,
C 07 D 317/08,
C 07 D 319/06,
C 07 D 319/12, C 07 F 7/10

(30) Priority: **17.12.87 ES 8800205**

(43) Date of publication of application:
**28.06.89 Bulletin 89/26**

(84) Designated Contracting States:
**AT BE CH DE ES FR GB GR IT LI LU NL SE**

(71) Applicant: **CENTRO MARGA PARA LA INVESTIGACION S.A.**
**Muntaner 212, 5o-510**
**E-08036 Barcelona (ES)**

(72) Inventor: **Ballester Rodés,Montserrat**
**Marqués de Santa Ana No 14, 2a**
**E-08023 Barcelona (ES)**

**Palomo Nicolau , Fransisco Eugenio**
**Dos de Mayo No 34, 2o-1a**
**E-08190 Sant Cugat del Vallés(Barcelona) (ES)**

**Palomo Coll, Antonio Louis**
**Escuelas Pias No 18, 5o-1a**
**E-08017 Barcelona (ES)**

(74) Representative: **Ballester Rodés, Albert**
**Muntaner 212, 5o- 508**
**E-08036 Barcelona (ES)**

Claims for the following Contracting States: ES + GR.

(54) **New-quanidino-thiazol compounds, their preparation, and use as intermediates of famotidine process.**

(57) New 2-guanidino-thiazol compounds with the general formulas

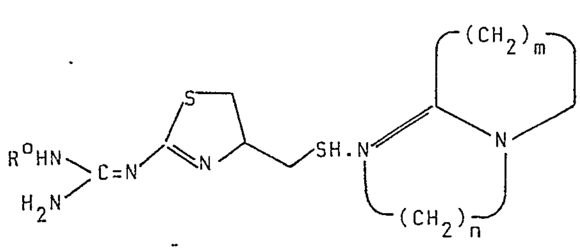

where R° represents an hydrogen atom or an alkyl group of low molecular weight, m = 2 to 7, n = 2 to 4, and

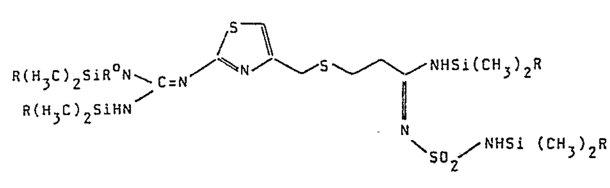

being R an alkyl group of low molecular weight and that may contain from one N-alkylsilyl group, that are important intermediates for the famotidine preparation, medically used as inhibitor of the gastric secretion.

EP 0 322 335 A1

## Description

### New 2-guanidino-thiazol compounds, their preparation, and use as intermediates of the famotidine process

The compound 2-guanidino-4-mercaptomethyl-thiazol, is of interest in the preparation of intermediates, whose application is oriented to obtain the N-sulfamyl-3- [(2-guanidinothiazol-4-yl)methylthio] -propionamidine, which is known by its gastric antisecretory properties.

The patent application ES 8700719, describes the reaction between 2-guanidino-4-chloromethyl-thiazol with sodium hydrogen sulfide just prepared. In the Patent JP 61 18,774 (CA, 105:60596v), the procedure consists in to realize in the presence of hydrogen sulfide the reaction between dicyanodiamide and 1,3-dimercaptoacetone; the latter formed from the 1,3- dichloroacetone with a salt of hydrogen sulfide. Those processes have the inconvenience that present the manipulation of the hydrogen sulfide, toxic and inflammable gas, and its later destruction, as it is liberated as a residual effluent. An important difficulty of those procedures, consists in the reaction mixture formed by the corresponding sulfide, disulfide and in the chloromethylthiazol case, its transformation into 4-methoxymethylthiazol. Also, are obtained compounds that are quantified by iodometry and that give negative the thiols test (active) with nitroprussiate. All of it causes bad yields and low purity.

Other processes propose the dihydrochloride of S-(2-guanidino-4- thiazolmethyl)isothiourea that by hydrolysis with sodium hydroxide yields the 2-guanidino-4-mercaptomethylthiazol, generated "in situ". This process of the isothiouronium salts has been described in several patents. For the preparation of 3-(2-aminothiazol-4-yl-methylthio)propionitrile in the GB 2052478A and 2055800A, the one of the 3-(2-guanidinothiazol-4-yl-methylthio)-propionitrile in EP 87,274 (GB 82/5075), ES 519,936 and EPO 128,736 (JP 83/102,206); for the derivatives of N-sulfamyl-propionamidine in US 4.496.737 and ES 540352.

The easy oxidation that experiments the thiolate generated in an alkaline medium of sodium hydroxide, originates the formation of disulfides and other impurities. The hydrolysis of the S-(2-guanidino-thiazol-4-yl-methyl)isothiourea dihydrochloride, proceeds at room temperature and more quickly by heating. In both cases, adjusting the PH by means of an acid, in relation to the sodium hydroxide used, it precipitates a viscous oil of brown color, of difficult manipulation, easily oxidable and insoluble in numerous organic solvents, in dichloromethane for example, making impossible an efficient isolation by extraction.

This operative way of generating "in situ" the thiolate, presents another inconvenient related to the impurities that contaminate the starting isothiouronium compound. All of them, in more or less quantity, shall be found in the final reaction product.

Are known by the experts the toxic, vesicant and irritant properties on the skin and mucous membranes of the compounds amidinothiourea, 1,3-dichloroacetone (carcinogen), and allergic reactions as the dermatitis that are produced in the manipulation with 2-guanidino-4-chloromethylthiazol. All these substances are contaminants that can be located in the isothiourea derivative that results in the reaction with chloromethyl-thiazol.

Besides the previous impurities that result from the reaction with the isothiouronium compound to obtain the 3-(2-guanidino-thiazol-4-yl-methylthio)propionitrile, other products are formed during the conversion into 3-(2-guanidino-thiazol-4-yl-methylthio)propionimidate.

The imidates preparation methods have been summarized by S.R. Sandler and W. Karo (Organic Functional Group Preparations, Academic Press, New York 1972) and R. Roger, D.G. Neilson (The Chemistry of Imidates; Chem . Rev. 61, pgs. 181, 183, 184; 1961). According to these authors, the moisture produces ester, the temperature originates the amide and the excess of alcohol forms ortoester and ammonium chloride. Also, from the experimental results it is questionable whether the use of diluents can be the cause of low yields and in some cases, it can be recommendable its addition to the reaction mixture after some days of reaction.

In the case of the 3-(2-guanidino-thiazol-4-yl-methylthio)propionitrile, its reaction with hydrogen chloride in methanol at 3°-6° C yields besides the imidate other compounds. Diluents as chloroform, dichloromethane or toluene are used, and operating with the molar ratio from 5 to 20 of hydrogen chloride per one mole of propionitrile, with 30 to 37 moles of methanol and double volume of the selected diluent. The results show that from 105,0g (95% assay) of 3-(2-guanidino-thiazol-4-yl-methylthio)propionitrile, are isolated about 90,0g of raw product. A subsequent treatment with dichloromethane, yielded by filtration 50,0g of imidate contaminated with ester and nitrile; from the evaporation to dryness of the dichloromethane were isolated about 40,0g of the ester.

In other experiments and depending on the molar concentration of hydrogen chloride at prolonged times, it was isolated a 25% of one non-identified compound of light cream color with a decomposition temperature of 220° C (high darkening) and amide with decomposition at 200° C.

Another undesirable behavior, is reflected in the reaction of the imidate with sulfamide, endangering the quality of the product with regard to the yield and the purity. Both characteristics are altered by diversity of competitive reactions as the formation of the propionamidine derivati ve, dimers of propionimidate-propionamidine and dimers with sulfamide bridge. The patent EPO 128,736, describes the reaction in methanol at 20°-30° C during three days; the isolated raw reaction product is recrystallized from dimethylformamide-water, and after from its aqueous solution as acetate, and by means of a base, the famotidine precipitates and a 48.8% yield is obtained. According to our results, in this way is prepared a polymorph of high melting point with a I.R.(KBr) registration in the region of 3400-3000 $cm^{-1}$ and with a characteristic X-ray diffraction

spectrum, that differ from the one of the low melting point polymorph.

By means of the procedure of the present invention, those problems are solved. It differs from the previous and from the described in the scientific and technical literature, by the utilization of new products and reagents for the reactions of beta-cyanoethylation, imidate formation and its conversion into sulfamide.

The process of the present invention comprises:

a)new salts of 2-guanidino-4-mercaptomethyl-thiazol with bicyclic amidines, preferably of 1,8-diazabicyclo [5.4.0] undec-7-ene (DBU) or 1,5-diazabicyclo [4.3.0] non-5-ene (DBN). These salts react instantaneously with acrylonitrile, with a virtually quantitative yield, being formed in aqueous medium the 3-substituted propionitrile derivative;

b)the new reagent oxonium hydrochloride, prepared from the hydrogen chloride-dioxane, for the formation in situ of the imidoyl chloride derived from the previous propionitrile, which with methanol "in situ", yields the corresponding propionimidate with an excellent selectivity; and

c)a new reaction with N,N′-bis-trimethylsilyl-sulfamide and the N-trimethylsilylimidate, at moderate temperatures in a non-hydroxylic solvent, preferably acetonitrile, dioxane, dimethylacetamide, dichloromethane or chloroform and from where, by distillation, the famotidine is obtained.

The present invention describes new 2-guanidinothiazol compounds and the use of new reagents, used as intermediates for the preparation of famotidine.

According to the invention, it provides one of the products of 2-guanidinothiazol with the general formula

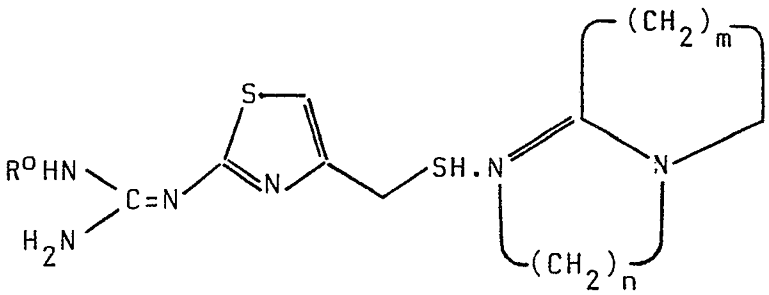

I

R° being an hydrogen atom or an alkyl group of low molecular weight, and m = 2 to 7 and n = 2 to 4.

By the term low molecular weight, it is meant a group having 1 to 5 carbon atoms in a linear or branched form. These groups can include one methyl, ethyl, isopropyl, terbutyl, etc.

Furthermore, the compound of general formula I, represents the basic salts of addition, that can exist in tautomeric forms.

The basic salts of addition include salts of the mercapto compound with organic bases of the amidines group where it can be m = 2 to 7 and n = 2 to 4. Preferably are selected the 1,8-diazabicyclo [5.4.0] undec-7-ene (DBU), 1,5-diazabicyclo [4.3.0] non-5-ene (DBN), etc.

A compound of formula I, is easily prepared by controlled hydrolysis of a S-(2-guanidino-thiazol-4-yl-methyl)amidino-isothiourea, S-isothiourea, S-(N-methyl)isothiourea and other derivatives described in our Spanish Application ES 8700719. The mentioned hydrolysis is realized in an inert solvent of the group that comprises the acetonitrile, dioxane, isopropanol, or their mixtures, in the presence of water and a bicyclic amidine, selected from among the DBN, DBU, etc.

The water is used in stoichiometric quantity or with an excess until a 10%, and the bicyclic amidine in an equimolecular ratio. In the case of using other inorganic or organic bases to neutralize the initial isothiouronium hydrochlorides, it is only necessary an equivalent of DBU, DBN, etc. Inorganic bases for that purpose, can be selected from the hydroxides, carbonates or bicarbonates of sodium or potassium, and the alkaline alcohoxides. Among the organic ones, the primary, secondary, tertiary and heterocyclic amines. Thus are preferred for example, triethylamine, diethylamine, tetramethylguanidine and pentamethylguanidine.

The formation of a salt with the formula I happens quickly and precipitates almost immediately from the reaction mixture. The conversion is virtually quantitative, the same as the selectivity, reaching a 95% isolation with respect to the theoretical. The reaction time can vary between 60 and 180 min. without exterior energy contribution.

By addition of acrylonitrile to the aqueous solution of a salt of formula I, a quick precipitation is produced of 3-(2-guanidino-thiazol-4-yl-methylthio)propionitrile that corresponds to an almost quantitative transformation. From the filtered aqueous liquids, the bicyclic amidine is recovered, to be recycled in the process.

Other compounds of this invention, can be produced from the 3-(guanidino-thiazol-4-yl-methylthio)propionitrile by means of its reaction with an oxonium salt, of the following formula:

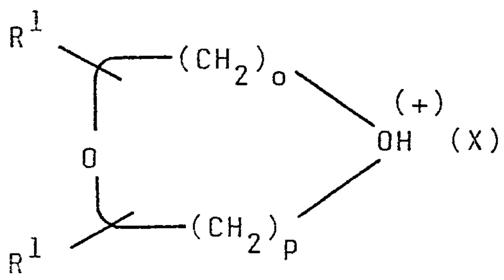

II

being X a chloride, bromide, methansulphonate or trifluoromethansulfonate, $R^1$ an hydrogen atom, methyl, ethyl or phenyl and o = 1-2, p = 2-3. For this purpose are adequate preferably the 1,3-dioxane or the 1,4-dioxane. In both cases an excess of dioxane is used to obtain a solution of the compound of formula II, with a 20% to 30% content of hydroxonium salt. Among those salts, are preferred the chloride, bromide or methansulphonate, that are suitably prepared from the dioxane and the corresponding acid.

An excess of dioxane is also used as diluent of the compound of formula II and for the formation, for instance, of the imidoyl halide of the following formula:

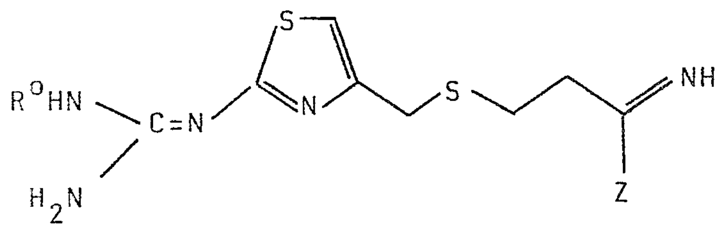

III

being Z a chlorine or bromine atom, and R° with the meaning previously given.

The compound of formula III, is not isolated and its formation in the presence of an adjusted quantity of methanol, yields the compound of formula III, being Z a methoxyl. In other cases, as described in our Spanish Application ES 8702202, the imidoyl chloride precipitates and its isolation is possible, although, due to the high activity, it is preferably to continue with the reaction to form the imidate. If these reactions are made in the presence of diluents, as the dichloromethane, chloroform or an excess of methanol, the formation of III is very slow and low yields are obtained.

The compounds of formula III, in the presence of ammonia or ammonium chloride, yield the amidine of formula III, where Z represents an amino group.

The molar ratio of the compound of formula II with respect to the 3-(2-guanidino-thiazol-4-yl-methylthio)propionitrile used, can vary from 3 to 7. The methanol content is adjusted to reach the dissolution of III. The time for the formation of the imidate comprises a period of 6 to 20 hours at the temperature of 3°C to 10°C. The starting 3-substituted-propionitrile can be indistinctly also in the form of hydrochloride or sulphate. The isolation is realized by means of conventional procedures.

The final step of the process is characterized because the compound of formula III, being Z a methoxyl, is reacted with a silylating agent to obtain the solution of a tautomer with the following formula :

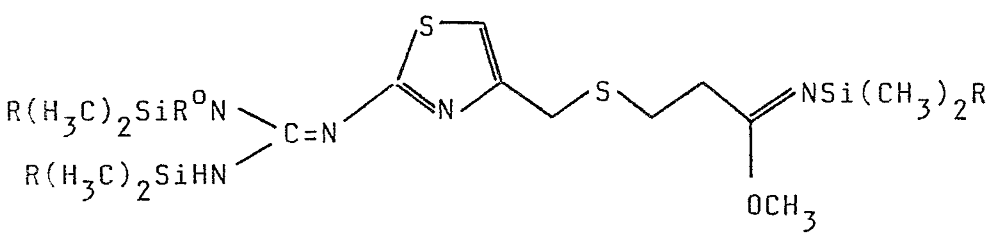

IV

where R° has the meaning previously given, and R an alkyl group having one to five carbon atoms linear or branched.

The compound of formula IV, is combined with a N-trialkyl silylsulfamide (VI) to obtain a solution of a

compound with the following formula:

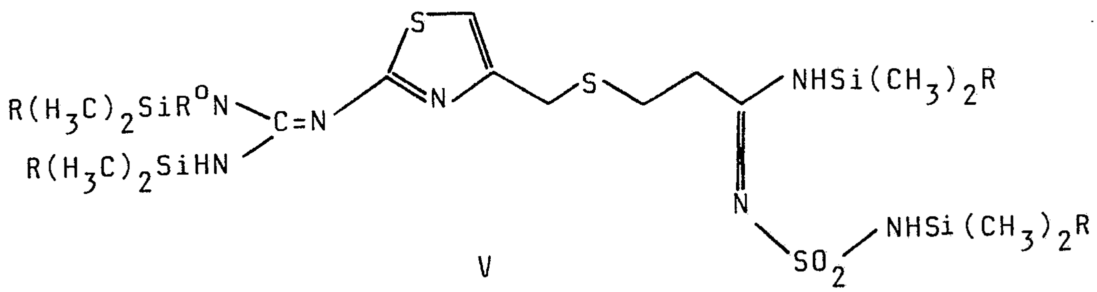

V

where R° and R have the meaning previously given, and representing V one of the possible tautomers.

Silylating agents adequate for the formation of the N-trialkylsilylimidate (IV) and a N-trialkylsilylsulfamide (VI) can be selected from the hexamethyldisilazane, 3-trimethylsilyl-2-oxazolidinone, 1,3-bis-trimethylsilylurea, N,O,bis-trimethylsilylacetamide, N-trimethylsilylacetamide, N-trimethylsilylsaccharin, N-trimethylsilylphtalimide, a trimethylsilylbiuret, N-trimethylsilylpyrrol, N-trimethylsilylimidazoline, trimethylchlorosilane, triethylchlorosilane, tripropylchlorosilane, dimethyl-terbuthylchlorosilane, N,N'-bis-trimethylsilylsulfamide, N,N'-tetrakistrimethylsilylsulfamide, N,N'-bis-dimethylterbuthylsilylsulfamide, N,O-bis-trimethylsulfamate, etc.

The compound VI can be a N-trialkysilylated derivative, preferably N-trimethylsilylated, mono-, di- or tri-substituted, depending on the quantity of silylating agent and of the trialkylsulfamide. From 1.5 to 4.0 equivalents of this product can be used per mole of imidate III, Z = methoxyl. The solvent is selected from the group constituted by the acetonitrile, dioxane, dichloromethane, chloroform or dimethylacetamide, and in general non-hydroxylic solvents, which are incompatible with the formation of the compounds of formula IV, V and VI.

In order to proceed to the de-silylation of V, the resulting solution of the reaction, is treated with the equivalent quantity of an alcohol selected from methanol, ethanol, isopropanol or n-buthanol, obtaining by stirring at room temperature, filtering and washing with methanol, the famotidine. The conversion is virtually quantitative, with a selectivity of 82% and an isolation yield of 70%. Proceeding according to our Spanish Application ES 8702020, it results the desired polymorph.

Example 1

1,8-diazabicyclo [5.4.0] undec-7-ene and 2-guanidino-4-mercaptomethyl-thiazol salt (DBU salt)

A)To the suspension of S-(2-guanidino-thiazol-4-yl-methyl)isothiourea hydrochloride (75.80 g; 0.25 mole) in acetonitrile (240 ml) and water (4.86 ml; 0.27 mole), under stirring, it is added all at once DBU (130.5 ml; 0.87 mole). It is produced a dissolution with elevation of the temperature from 20° C to 47° C. Almost immediately, an abundant precipitate is formed an the mixture is kept under stirring during some time (120-180 min., depending on the efficacy of the stirring), letting it reach the room temperature. The product is filtered, washed with acetonitrile and later dichloromethane (or isopropanol); dried in vacuum it yields the title's compound, 76.6 g to 80.9 g with a yield of 90% to 95% and m.p. = 152-155° C (d) (darkening at 140° - 145° C). Positive test with solution of nitroprussiate and purity from 95.0 to 98.0% (titrated by iodometry). I.R.(KBr) $\nu cm^{-1}$: intense and wide registration from 3400 to 2200 with sub-bands and centered at about 3000, registrations at 1630(s), 1570(s), 1450(s) and 1210(s).

Microanalysis ($C_{14}H_{24}N_6S_2$; M.W. = 340.50) : Calculated : C, 49.3; H, 7.1; N, 24.7; S, 18.8. Found : C, 49.0: H, 7.2; N, 24.5; S, 19.0.

Replacing the previous isothiourea by the equivalent quantity of S-(2-guanidino-thiazol-4-yl-methyl)-N-methylisothiourea dihydrochloride, it results the title's compound with identical characteristics and a similar yield.

Replacing the acetonitrile by isopropanol (100 ml), after the addition of the DBU a supersaturated solution is produced, from where inoculating with DBU salt at the temperature of 32° C approx., the precipitation begins quickly. The title's compound is isolated with a 75% yield.

B) Following the example 1A and replacing the isothiourea derivative by S-(2-guanidino-thiazol-4-yl-methyl)amidinoisothiourea hydrochloride (86.30 g; 0.25 mole), it results the title's compound with identical characteristics and a similar yield. The amidinoisothiouronium (dihydrochloride) is prepared by reaction of 1,3-dichloroacetone with two equivalents of amidinoisothiourea in ethanol, and alternatively from 2-guanidino-4-chloromethyl-thiazol reaction with an equivalent of amidinoisothiourea, heating the mixture in isopropanol during 60 min. In both cases, by elimination of the alcohol, it results the isothiouronium compound, (semi-solid), which can be used without previous purification.

Microanalysis. S-(2-guanidino-thiazol-4-yl-methyl)amidinoisothiourea dihydrochloride. (M.W. = 345,26; $C_7H_{12}N_8S_2$. 2HCl) : Calculated : C. 24.3; H, 4.2; N, 32.4; S, 18.6; Cl, 20.5 Found : C, 24.5; H, 4.0; N, 32.9; S, 18.9; Cl, 20.9.

Replacing the previous isothiouronium derivative by the xanthate, the title's compound is obtained with

similar characteristics.

C) To the solution of 2-guanidino-4-mercaptomethyl-thiazol (18.82 g; 0.1 mole) in acetonitrile (75 ml), DBU (16.7 ml; 0.11 mole) is added. Immediately, a precipitate of the DBU salt is produced. After some time (30 min.), under stirring at room temperature, it is filtered, washed successively with acetonitrile (25 ml) and dichloromethane (25 ml). It results the title's compound with a virtually quantitative yield and identical characteristics to the product of the example 1A.

To the product of the reaction between 1,3-dimercaptoacetone and an equivalent of dicyandiamide (Pat. JP 61 18,774) in acetonitrile, DBU is added, causing the precipitation of the title's compound.

Example 2

1,5-diazabicyclo [4.3.0] non-5-ene and 2-guanidino-4-mercaptomethyl-thiazol salt (DBN salt)

To the suspension of dihydrochloride of S-(2-guanidino-4thiazolylmethyl)isothiourea dihydrochloride (12.13 g; 0.04 mole) in acetonitrile (30 ml), under stirring, it is added successively water (0.81 ml, 0.045 mole) and DBN (16.2 ml; 0.13 mole). The initial temperature (25°C) reaches about 45°C resulting nearly a complete dissolution, from where the precipitation begins quickly. The mixture is stirred during 120 to 180 min., letting the temperature go down (20°C). It is filtered, washed with acetonitrile and dichloromethane; dried at reduced pressure yields 11.80 g (Yld.= 94.5%) of the title's compound with m.p. = 127-129°C. I.R.(KBr) ν $c^{-1}$ : wide registrations from 3500 to 2600 with sub-bands, register with amplitude from 1700 to 1500 with bands at 1665(s), 1630(w), 1600(m) and 1530(s).

Microanalysis. ($C_{12}H_{20}N_6S_2$; M.W. = 312.45) : Calculated: C, 46.1; H, 6.4; N, 26.9; S, 20.5. Found : C, 46.3; H, 6.5; N, 27.0; S, 20.2.

This salt is transformed as time goes on, giving negative the nitroprussiate test; in the presence of an equivalent of sodium hydroxide and acrylonitrile, it yields after some time (150 min.) the corresponding propionitrile derivative.

Example 3

3-(2-guanidino-thiazol-4-yl-methylthio)propionitrile

A)A recently prepared DBU salt is dissolved according to the example 1 (68.2 g or 74.1 g of 92% resulting from the washing with dichloromethane, 0.20 mole) in water (300 ml). Cooling with an ice-water bath, the reaction temperature is controlled at 20-25°C and acrylonitrile (16.50ml, 0.25 mole) is gradually added in 5 min. From the mixture, under stirring, a white precipitate is produced almost immediately. The end of the reaction coincides practically with the total addition of the acryionitrile, the test of nitroprussiate being negative. It is stirred during 15 min., cooling (15°C-20°C), it is filtered, washed with water and dried, yielding 45.0 g (Yld. 93.3%) of the title's compound with m.p. = 127-129°C (recrystallized, m.p. =132°C). I.R.(KBr) $cm^{-1}$ : 3420(s), 3230(w), 3080(m), 2220(m), 1630(s), 1590(s), 1535(s) and 1450(s).

B)Following the example 3A, the DBU salt is substituted by DBN salt (example 2, 70.0 g of 90%; recently prepared and proceeding from the washing). The reaction with acrylonitrile yields a mixture that is adjusted at PH = 8.0-8.5 with 36% hydrogen chloride (0.20 mole approximately). The precipitate, isolated and dried corresponds to the title's compound with identical characteristics and a similar yield.

Example 4

3-(2-Guanidino-thiazol-4-yl-methyl-thio)propionimidoylchloride-hydrochloride

To the 1,4-dioxane hydrochloride (100.0 g, HCl 25% w/w; 0.68 mole), at room temperature (20°C-25°C), it is added in three portions the compound 3-(2-guanidino-thiazol-4-yl-methylthio)propionitrile (41.02 g; 0.17 mole). It is stirred during some time (120 min.) and afterwards it is cooled at 0-5°C. It results a mixture of pasty product, corresponding to the title's compound.

Example 5

Methyl-3-(2-guanidino-thiazol-4-yl-methylthio)propionimidate

A)To the mixture of the example 4, cooled at 0-5°C, with energetic stirring, anhydrous methanol is gradually added (40 ml, approx. 0.1 mole), controlling the previous temperature. It results a solution, that is stirred at 5°C during some time (8-20 hours). Next, it is gradually added to a cold mixture (0-10°C) formed by a solution of sodium hydroxide (13.3 g; 97%; 0.33 mole) and anhydrous sodium carbonate (37.1 g; 0.35 mole) in water (700 ml) and dichloromethane (300 ml). After some time (60 min.) under stirring at 10°C, it is filtered, washed successively with abundant water (500 ml), acetonitrile and dichloromethane. Dried, it results the title's compound, 37.2 g with a 80% Yield and m.p. = 130-135°C (suspended while it is hot in acetonitrile m.p. = 136-138.5°C).

From the decanted organic phase, dried with anhydrous sodium sulphate, and the solvent evaporated, another portion of the raw product is recovered, that washed with dichloromethane and dried, is recycled in the process.

B)Following the examples 4A and 5A, replacing the 1,4-dioxane by 1,3-dioxane, the respective compounds

of the title are obtained with similar yield and characteristics.

C)1,4-dioxane hydrochloride (42.0 g at 30% w/w; 0.34 mole) is cooled at 0-5°C. It is gradually added anhydrous methanol (20 ml; 0.1 mole approx.) and afterwards in two portions, 3-(2-guanidino-thiazol-4-yl-methylthio)propionitrile (20.51 g; 0.085 mole), controlling the previous temperature. It results a solution that is stirred during 20 hours at 5°C. For the isolation of the imidate, it is proceeded as described in the example 5A, adding dichloromethane (300 ml) to the alkaline aqueous solution. The mixture is stirred energetically. It results the title's compound with a 82% yield and similar characteristics. From the dichloromethane organic phase, some product is recovered that is recycled.

D)Following the examples 4A and 5A, substituting the 1,4-dioxane by 2-methyl-, 2-ethyl- or 2-phenyl-dioxane, identical results are obtained.

Example 6

3-(2-Guanidino-thiazol-4-yl-methylthio)propionamidine

At 45°C it is heated a mixture of methyl-(2-guanidino-thiazol-4-yl-methylthio)propionimidate (2.73 g; 0.01 mole), ammonium chloride (1.12 g; 0.021 mole), methanol (2.5 ml) and acetonitrile (10 ml). Afterwards it is let cool at room temperature and stirred for a time (24 hours). The ammonium chloride is filtered (0.58 g; 1.09 mole). To the liquids an equivalent of hydrogen chloride-dioxane is added, precipitating an oil that left in the refrigerator, solidifies. Crystallized in ether (by evaporation), m.p. = 183-187°C.

Example 7

Solutions of N,N-trimethylsilyl-methyl-3-(2-guanidinothiazol-4-yl-methylthio)propionimidate

A)3-Trimethylsilyl-2-oxazolidinone: To the suspension of methyl-3-(2-guanidino-thiazol-4-yl-methylthio)propionimidate (methyl-imidate, 27.3 g; 0.1 mole) in acetonitrile (80 ml), it is added 3-trimethylsilyl-2-oxazolidinone (50.5 ml; 0.33 mole) and with stirring it is heated gradually at 65-70°C. After some time (15 min.) is cooled at room temperature resulting a solution of the title's compound.

B)Trimethylchlorosilane: A suspension of the methylimidate (27.3 g; 0.1 mole) in dichloromethane (200 ml) with triethylamine (42.0 ml; 0.30 mole) and trimethylchlorosilane (34.76 g; 0.32 mole), is stirred at the temperature of 30°C. After a few time, it results a solution of the title's compound.

Replacing the silylating agent by trimethylsilylimidazolidinone, the same result is obtained.

C)N,N′-tetrakis-trimethylsilylsulfamide: A mixture of the methyl-imidate (27.3 g; 0.1 mole) and N,N′-tetrakis-trimethylsilylsulfamide (38.4 g; 0.1 mole) in anhydrous dimethylacetamide (80 ml), is stirred at the temperature of 40°C ,resulting a quick solution. It is cooled at 33°C in a short time (10 min.), and next at room temperature. A title's compound solution is obtained.

Replacing the silylating agent by trimethylsilylphtalamide, the same result is obtained.

D)N,N′-Bis-trimethylsilylacetamide: The suspension of the methyl-imidate (27.3 g; 0.1 mole) in chloroform (100 ml) is treated with N,N′-bis- trimethylsilylacetamide (65.0 g; 0.32 mole) under stirring and heating. In a few time results a solution of the title's compound. Replacing the silylating agent by hexamethyldisilazane, the same result is obtained.

Example 8

Solution of a N-trimethylsilyl-N-sulfamyl-3-(2-guanidino-thiazol-4-yl-methylthio)-propionamidine

The suspension of methyl-3-(2-guanidino-thiazol-4-yl-methylthio)propionimidate (10.94 g; 0.04 mole) and N,N′-bis-trimethyl-silylsulfamide (28.85 g; 0.12 mole) in acetonitrile (32 ml), is heated slowly (about 45 min.) until 55-60°C, resulting a complete solution. It is let to reach the room temperature. After 70 hours under stirring at 20-25°C, it is confirmed by thin layer chromatography, the absence of imidate and the formation of the title's compound.

Example 9

Solution of a N-trimethylsilyl-N-sulfamyl-3-(2-guanidino-thiazol-4-yl-methylthio)propionamidine: starting from example 7

To the solution obtained according to the example 7C, sulphamide (19.2 g; 0.2 mole) is added. It is stirred during 24 hours, resulting a solution of the title's compound. By thin layer chromatography is controlled the disappearance of the methyl-imidate and the formation of the sulfamyl derivative.

Example 10

De-silylation procedure: Famotidine isolation

A) To the reaction product of the example 8, methanol (9.0 ml; 0.22 mole) is added slowly and under stirring. After adding the first drops, a little precipitate that retains impurities is originated. The colorless solution is decanted, continuing with the addition of the alcohol, that produces the separation of an oil that is decanted,

mixes with acetonitrile (30 ml) and is kept under stirring, solidifying. Filtered, washed successively with acetonitrile and dichloromethane, it yields 9.45 g (70% raw yield) of the title's compound. The I.R.(KBr) spectrum shows the characteristics registration at: 3480, 3360 with sub-bands at 3380 and 3340, 3210, 3080 and 2910 $cm^{-1}$, corresponding to the low melting point polymorph.

B) The reaction product of the example 9 and after the addition of isopropanol (24.0 g; 0.4 mole) is diluted with dichloromethane (250 ml), and cooling in an ice-water bath, the mixture is kept under stirring. The inoculation with famotidine facilitates the solidification. Filtered, washed with dichloromethane and dried, the title's compound is isolated with a yield similar to the example 10A one.

C) The solution obtained according to the example 8, is treated with methanol (25 ml) and, next, the solvent is distilled at reduced pressure till a volume of about 25 ml. It is stirred at room temperature, precipitating a solid that is filtered, washed with acetonitrile and dichloromethane, resulting the title's compound with a similar yield.

D) The famotidine hydrochloride, isolated from methanol at PH = 2.0, suspended again in the alcohol and adjusted at pH = 7.5 with triethylamine, yields polymorph with m.p. = 165-173°C; I.R.(KBr), characteristics registrations in the region 3500-3000 : 3400 with sub-bands at 3430 and 3390, 3330, 3220, and 3070 $cm^{-1}$, and with X-ray diffraction spectrum identical as the described in the example 10E.

E) The solution of 5.0 g of famotidine in dimethylacetamide (10.0 ml), is gradually added in dichloromethane (60.0 ml) at room temperature and with energetic stirring. The solidified precipitate , is stirred 15 min., filtered, washed and dried yielding 4.90 g with a m.p. = 168-172°C (polymorph with a high melting point, in the shape of concretions). I.R. (KBr) $\nu cm^{-1}$ : characteristic registrations at 3400 (doublet at 3430 and 3390), 3300, 3220 and 3070.

X-ray diffraction spectrum, at $\lambda$ = 1.5405 Å using a radiation source of Cu, 40Kw and 20 ma, coming from a Siemens Kristalloflex 810 Interface DACO-Mp equipment, with the following characteristics :

| d = spacing | $I/I_1$ = relative intensities |
|---|---|
| 20.23 | 0.05 |
| 8.22 | 0.14 |
| 6.55 | 0.12 |
| 6.24 | 0.14 |
| 6.09 | 0.26 |
| 5.88 | 0.13 |
| 5.57 | 0.08 |
| 5.14 | 0.12 |
| 4.72 | 0.27 |
| 4.53 | 0.19 |
| 4.45 | 1.00 |
| 4.36 | 0.69 |
| 4.24 | 0.55 |
| 4.12 | 0.55 |
| 3.93 | 0.17 |
| 3.79 | 0.34 |
| 3.70 | 0.22 |
| 3.58 | 0.25 |
| 3.47 | 0.09 |
| 3.43 | 0.14 |
| 3.33 | 0.40 |
| 3.27 | 0.14 |
| 3.14 | 0.24 |
| 3.06 | 0.17 |
| 3.02 | 0.18 |
| 2.95 | 0.05 |
| 2.79 | 0.11 |
| 2.74 | 0.10 |
| 2.67 | 0.10 |
| 2.62 | 0.06 |
| 2.58 | 0.09 |
| 2.51 | 0.04 |
| 2.47 | 0.06 |

| | |
|---|---|
| 2.36 | 0.09 |
| 2.33 | 0.04 |
| 2.30 | 0.06 |
| 2.28 | 0.12 |
| 2.24 | 0.07 |
| 2.22 | 0.05 |
| 2.13 | 0.13 |
| 2.10 | 0.04 |
| 2.01 | 0.04 |
| 1.97 | 0.04 |
| 1.95 | 0.04 |
| 1.83 | 0.03 |
| 1.81 | 0.05 |
| 1.77 | 0.07 |
| 1.73 | 0.04 |
| 1.71 | 0.04 |
| 1.68 | 0.07 |

**Claims**

1. Compounds of 2-guanidinothiazol, its preparation and utilization as intermediates of the famotidine process, characterized by :

a)A new compound of general formula

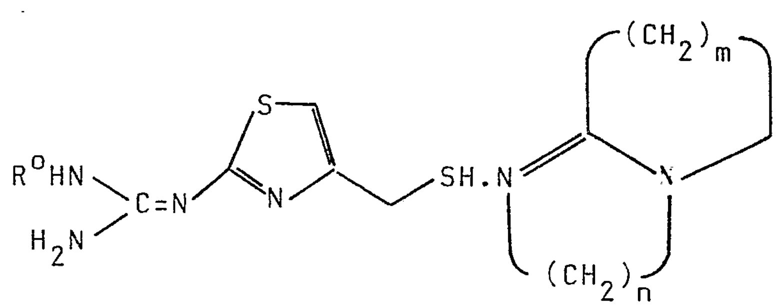

I

where R° represents an hydrogen atom or a $C_1$-$C_5$ alkyl group, m = 2 to 7 and n = 2 to 4, that is reacted with acrylonitrile to obtain 3-(2-guanidino-thiazol-4-yl-methylthio)propionitrile,

b)a new hydroxonium salt of the general formula

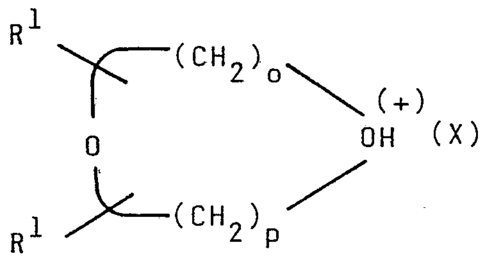

II

where $R^1$ can represent an hydrogen atom, a methyl, an ethyl or a phenyl, o = 1 to 2, p = 2 to 3, and X a chloride or bromide ion, methansulphonate or trifluoromethansulphonate, that is reacted with the 3-substituted-propionitrile derivative of I during 8-20 h., to obtain a compound with the general formula

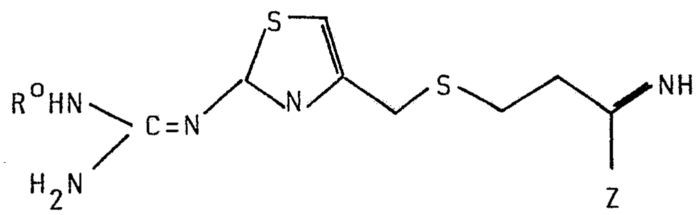

III

where R° is as hereinbefore defined and Z is an halogen atom, and that in the presence of methanol it results a compound of the formula III, being Z a methoxyl group, and that with ammonia or ammonium chloride yields a compound of the formula III, Z being an amino group; they can be obtained in the form of a salt of addition of an acid represented in formula II by X,

c)a new silyl-compound with the general formula

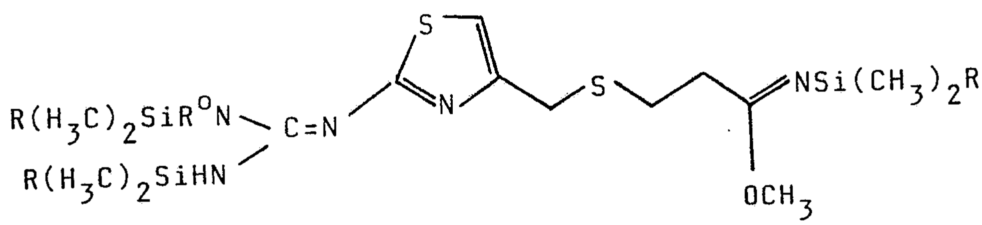

IV

where R° is as hereinbefore defined, R can be a $C_1$-$C_4$ alkyl group, linear or branched, that is obtained from the reaction of a compound of formula III and a silylating agent selected from a N-silylamine, N-silylamide, N-silylsulfamide or an halosilane with $C_1$-$C_4$ alkyl groups,

d)a new silyl-compound with the general formula

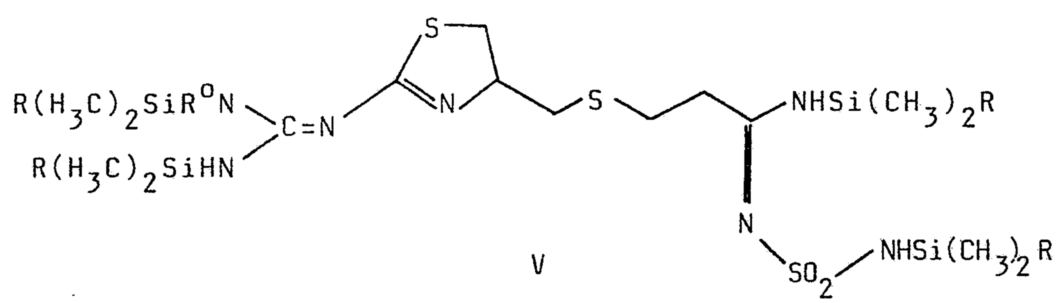

V

where R° and R are as hereinbefore defined, which is obtained from the reaction at temperatures of 20 to 60° C, of a compound of formula III or IV with a sulfamide of the following formula

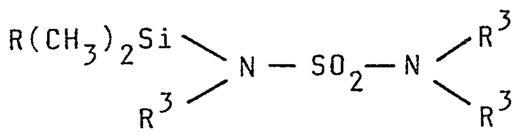

VI

where R is as hereinbefore defined and $R^3$ can be an hydrogen atom, a group $R(CH_3)_2Si$, or a $C_1$-$C_4$ alkyl silyl,

e) a silyl-compound of the formula V, where $R^0$ represents an hydrogen atom, that is reacted at room temperature with a $C_1$-$C_5$ alcohol, linear or branched, to obtain famotidine.

2. A process according to claim 1, characterized because a compound of formula I, being $R^0$ an hydrogen atom, is prepared from a compound selected from a S-(2-guanidino-thiazol-4-yl-methyl) derivative of isothiourea, N-methylisothiourea, amidinoisothiourea or a xanthate, which in a solvent selected from the acetonitrile, isopropanol or its mixtures, is reacted at room temperature with an equivalent of water, an inorganic base as the sodium or potassium hydroxides, an organic base as the triethylamine, diethylamine or a non-cyclic amidine as the tetramethylguanidine or pentamethylguanidine or a bicyclic amidine.

3. A process according to the first claim, characterized because a compound of formula I, being $R^0$ an hydrogen atom, is prepared, at room temperature, making to react 2-guanidino-4-mercaptomethyl-thiazol in a solvent selected from the acetonitrile, the isopropanol or their mixtures, with an equivalent of a bicyclic amidine.

4. A process according to the first claim, characterized because the salt of formula I, is selected from the salts of the amidines 1,8-diazabicyclo [5.4.0] undec-7-ene and 1,5-diazabicyclo [4.3.0] non-5-ene.

5. A process according to the first claim, characterized because an hydroxonium salt of formula II, where R represents an hydrogen atom, o = 1, p = 3 or o = p = 2, is selected from the 1,3-dioxane or 1,4-dioxane, and being X = chloride or bromide, it results from the reaction of the dioxane with hydrogen chloride or bromide, that at the concentration of 20%-30% is combined with 3-(2-guanidino-thiazol-4-yl-methylthio)propionitrile in the molar ratio hydroxonium salt:propionitrile comprised from 1:3 to 1:7, during a time of 6 to 20 h. and temperature of 3°-10° C, to obtain a compound of formula III, being R° an hydrogen atom, Z a chlorine or bromine atom, that by reaction with methanol in the molar ratio 1:4 to 1:7, yields the compound of formula III, being Z a methoxyl group.

6. A process according to the first claim, characterized because a compound of formula III, $R^0$ being an hydrogen atom, or its addition salt of an acid selected from the sulphate, methansulphonate, trifluoromethansulphonate, chloride or bromide, is reacted with a silylating agent selected from the group constituted by hexamethyldisilazane, 3-trimethylsilyl-2-oxazolidinone, 1,3-bis-trimethylsilylurea, N,O-bis-trimethylsilylacetamide, N-trimethylsilylacetamide, N-trimethylsilylsaccharin, N-trimethylsilylphtalimide, a N-trimethylsilylbiuret, N-trimethylsilylpirrol, N-trimethylsilylimidazoline, trimethylchlorosilane, triethylchlorosilane, tripropylchlorosilane, dimethylterbuthylchlorosilane, N,O-bis-trimethylsilylsulfamate, to obtain a solution of the silyl-compound of formula IV, which by reaction with a N-trimethylsilylsulfamide yields the compound of formula V, where R may be a $C_1$-$C_4$ alkyl group.

7. A process according to the first claim, characterized because a compound of the formula III or its addition salt, being $R^0$ an hydrogen atom, is reacted with a compound of the formula VI, selected from the N,N′-bis-trimethylsilylsulfamide, N,N′-tetrakis-trimethylsilylsulfamide, N, N′- bis-dimethyl-terbutylsilylsulfamide, N,N′-bis-triethylsilylsulfamide, N,N′-bis-tripropylsilylsulfamide, to obtain a solution of a silyl-compound of the formula V where R° represents an hydrogen atom and R has the meaning previously given, in a inert solvent non hydroxylic, preferably selected from the acetonitrile, dioxane, dimethylacetamide, dichloromethane or chloroform.

8. A process according to the first claim, characterized because a solution of the compound of the formula V, being $R^0$ an hydrogen atom and R a methyl group, is reacted with an alcohol selected from the methanol, isopropanol or n-butanol to obtain famotidine.

9. New 2-guanidinothiazol compounds, their preparation and utilization as intermediates of the famotidine process.

**Claims for the following Contracting States: E; GR.**

1. New process for the preparation of famotidine, characterized by:

a) A new compound of general formula

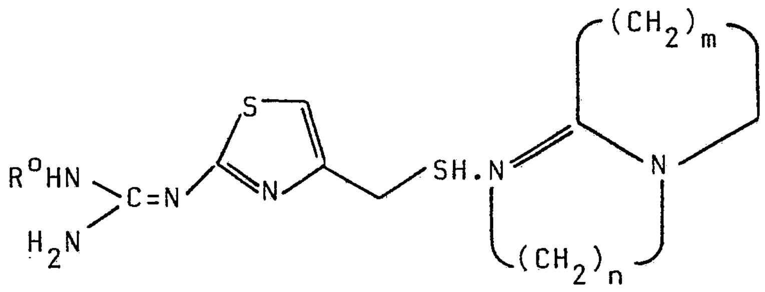

I

where R° represents an hydrogen atom or a $C_1$-$C_5$ alkyl group, m = 2 to 7 and n = 2 to 4, that is reacted with acrylonitrile to obtain 3-(2-guanidino-thiazol-4-yl-methylthio)propionitrile,

b)a new hydroxonium salt of the general formula

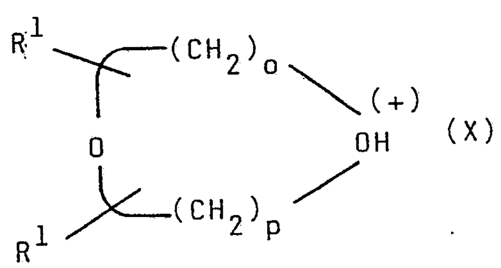

II

where $R^1$ can represent an hydrogen atom, a methyl, an ethyl or a phenyl, o = 1 to 2, p = 2 to 3, and X a chloride or bromide ion, methansulphonate or trifluoromethansulphonate, that is reacted with the 3-substituted-propionitrile derivative of I during 8-20 h., to obtain a compound with the general formula

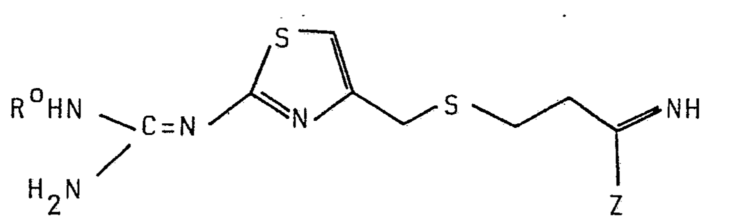

III

where R° is as hereinbefore defined and Z is an halogen atom, and that in the presence of methanol it results a compound of the formula III, being Z a methoxyl group, and that with ammonia or ammonium chloride yields a compound of the formula III, Z being an amino group; they can be obtained in the form of a salt of addition of an acid represented in formula II by X,

c)a new silyl-compound with the general formula

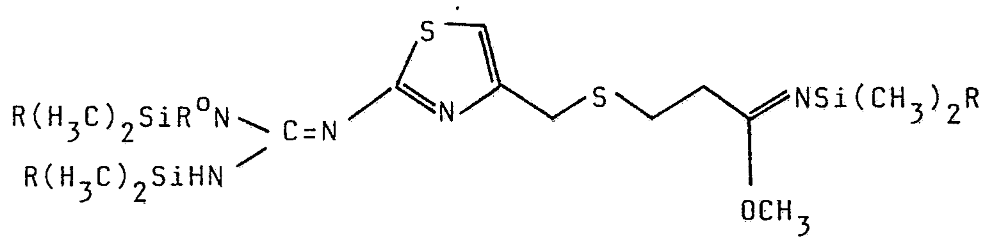

IV

where R° is as hereinbefore defined, R can be a $C_1$-$C_4$ alkyl group, linear or branched, that is obtained from the reaction of a compound of formula III and a silylating agent selected from a N-silylamine, N-silylamide, N-silylsulfamide or an halosilane with $C_1$-$C_4$ alkyl groups,

d)a new silyl-compound with the general formula

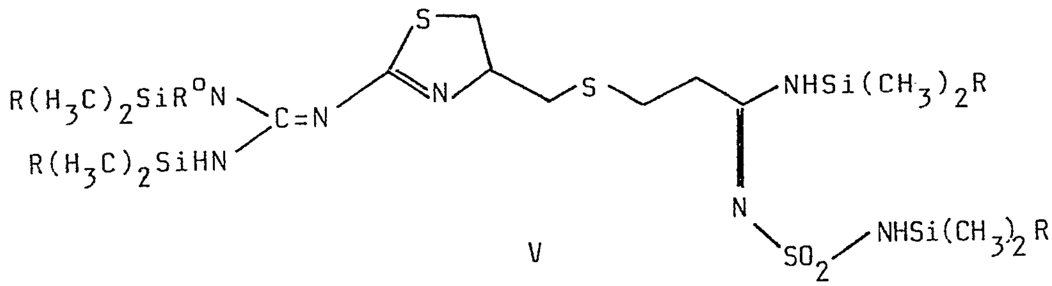

V

where R° and R are as hereinbefore defined, which is obtained from the reaction at temperatures of 20 to 60° C, of a compound of formula III or IV with a sulfamide of the following formula

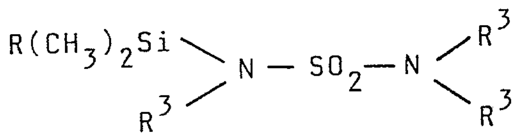

VI

where R is as hereinbefore defined and $R^3$ can be an hydrogen atom, a group $R(CH_3)_2Si$, or a $C_1$-$C_4$ alkyl silyl,

e) a silyl-compound of the formula V, where $R^0$ represents an hydrogen atom, that is reacted at room temperature with a $C_1$-$C_5$ alcohol, linear or branched, to obtain famotidine.

2. A process according to claim 1, characterized because a compound of formula I, being $R^0$ an hydrogen atom, is prepared from a compound selected from a S-(2-guanidino-thiazol-4-yl-methyl) derivative of isothiourea, N-methylisothiourea, amidinoisothiourea or a xanthate, which in a solvent selected from the acetonitrile, isopropanol or its mixtures, is reacted at room temperature with an equivalent of water, an inorganic base as the sodium or potassium hydroxides, an organic base as the triethylamine, diethylamine or a non-cyclic amidine as the tetramethylguanidine or pentamethylguanidine or a bicyclic amidine.

3. A process according to the first claim, characterized because a compound of formula I, being $R^0$ an hydrogen atom, is prepared, at room temperature, making to react 2-guanidino-4-mercaptomethyl-thiazol in a solvent selected from the acetonitrile, the isopropanol or their mixtures, with an equivalent of a bicyclic amidine.

4. A process according to the first claim, characterized because the salt of formula I, is selected from the salts of the amidines 1,8-diazabicyclo [5.4.0] undec-7-ene and 1,5-diazabicyclo [4.3.0] non-5-ene.

5.A process according to the first claim, characterized because an hydroxonium salt of formula II, where R represents an hydrogen atom, o = 1, p = 3 or o = p = 2, is selected from the 1,3-dioxane or 1,4-dioxane, and being X = chloride or bromide, it results from the reaction of the dioxane with hydrogen chloride or bromide, that at the concentration of 20%-30% is combined with 3-(2-guanidino-thiazol-4-yl-methylthio)propionitrile in the molar ratio hydroxonium salt:propionitrile comprised from 1:3 to 1:7, during a time of 6 to 20 h. and temperature of 3°-10° C, to obtain a compound of formula III, being R° an hydrogen atom, Z a chlorine or bromine atom, that by reaction with methanol in the molar ratio 1:4 to 1:7, yields the compound of formula III, being Z a methoxyl group.

6. A process according to the first claim, characterized because a compound of formula III, R° being an hydrogen atom, or its addition salt of an acid selected from the sulphate, methansulphonate, trifluoromethansulphonate, chloride or bromide, is reacted with a silylating agent selected from the group constituted by hexamethyldisilazane, 3-trimethylsilyl-2-oxazolidinone, 1,3-bis-trimethylsilylurea, N,O-bis-trimethylsilylacetamide, N-trimethylsilylacetamide, N-trimethylsilylsaccharin, N-trimethylsilylphtalimide, a N-trimethylsilylbiuret, N-trimethylsilylpirrol, N-trimethylsilylimidazoline, trimethylchlorosilane, triethylchlorosilane, tripropylchlorosilane, dimethylterbuthylchlorosilane, N,O-bis-trimethylsilylsulfamate, to obtain a solution of the silyl-compound of formula IV, which by reaction with a N-trimethylsilylsulfamide yields the compound of formula V, where R may be a $C_1$-$C_4$ alkyl group.

7. A process according to the first claim, characterized because a compound of the formula III or its addition salt, being $R^0$ an hydrogen atom, is reacted with a compound of the formula VI, selected from the N,N′-bis-trimethylsilylsulfamide, N,N′-tetrakis-trimethylsilylsulfamide, N, N′- bis-dimethyl-terbutylsilylsulfamide, N,N′-bis-triethylsilylsulfamide, N,N′-bis-tripropylsilylsulfamide, to obtain a solution of a silyl-compound of the formula V where R° represents an hydrogen atom and R has the meaning previously given, in a inert solvent non hydroxylic, preferably selected from the acetonitrile, dioxane, dimethylacetamide,

dichloromethane or chloroform.

8. A process according to the first claim, characterized because a solution of the compound of the formula V, being R° an hydrogen atom and R a methyl group, is reacted with an alcohol selected from the methanol, isopropanol or n-butanol to obtain famotidine.

European Patent Office

# EUROPEAN SEARCH REPORT

Application number: EP 88500031.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - A1 - 3 033 169 (BRISTOL MYERS CO.)<br>* Claims 5,26 *<br>-- | 1,2 | C 07 D 277/18<br>C 07 D 487/04<br>C 07 D 471/04<br>C 07 D 317/08<br>C 07 D 319/06<br>C 07 D 319/12<br>C 07 F 7/10 |
| X | RÖMPPS CHEMIE-LEXIKON, edition 8, vol. 2, 1981<br>FRANCKH'SCHE VERLAGSBUCHHANDLUNG, Stuttgart<br>pages 975-976<br>* Page 975, column 2, 4th line from the bottom *<br>---- | 1b) | |

| TECHNICAL FIELDS SEARCHED (Int. Cl.4) |
|---|
| C 07 D 487/00 |
| C 07 D 471/00 |
| C 07 D 317/00 |
| C 07 D 319/00 |
| C 07 D 277/00 |
| C 07 F 7/00 |

The present search report has been drawn up for all claims

| Place of search | Date of completion of the search | Examiner |
|---|---|---|
| VIENNA | 15-02-1989 | BRUS |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document